Europäisches Patentamt

European Patent Office

Office européen des brevets

(11) Publication number: **0 335 635**
A1

(12) **EUROPEAN PATENT APPLICATION**

(21) Application number: 89303004.9

(22) Date of filing: 28.03.89

(51) Int. Cl.⁴: **C 12 N 15/00**
C 07 H 21/04, A 61 K 39/21

(30) Priority: 28.03.88 US 174005

(43) Date of publication of application:
04.10.89 Bulletin 89/40

(84) Designated Contracting States:
AT BE CH DE ES FR GB GR IT LI LU NL SE

(71) Applicant: **THE BOARD OF TRUSTEES OF THE LELAND STANFORD JUNIOR UNIVERSITY**
**Encina 105 Stanford University**
**Stanford California 94305 (US)**

(72) Inventor: **McCune, Joseph McCrary**
**51 Clipper**
**San Francisco California 94114 (US)**

(74) Representative: **Harrison, David Christopher et al**
**MEWBURN ELLIS & CO 2/3 Cursitor Street**
**London EC4A 1BQ (GB)**

(54) Mutated HIV envelope protein.

(57) Novel DNA constructs and expression products are provided related to the HIV virus, where the gp160 encoding sequence is mutated to prevent cleavage to the mature protein product. The mutated protein and the virion containing the mutated protein may serve as vaccines to protect a host against infection and in the study of the maturation of gp160 and methods and compositions for inhibiting the maturation.

EP 0 335 635 A1

**Description**

## MUTATED HIV ENVELOPE PROTEIN

The field of this invention concerns vaccines for AIDS virus, modification of AIDS virus, and a probe useful in finding inhibitors of cellular proteases necessary for the maturation of the AIDS virus.

The AIDS virus, (Human Immunodeficiency Virus - HIV) has been considered a major threat to populations throughout the world. For the most part, the response has been directed to educational and to prophylactic measures, to reduce the transmission of the virus. A large amount of energy has been spent in investigating the virus, by sequencing the virus, isolating the various proteins, investigating the nature of the immune response to these proteins, the genes involved with replication, and the like. Despite the enormous effort expended on the various characteristics of the virus and its mechanism for infection, there still remains substantial uncertainties about the virus.

Because of the mortal nature of the virus, there have been numerous investigations in an effort to provide immune protection against the virus. A number of viral protein fragments have been suggested as vaccines. In addition, more recently, peptides associated with the CD4 sequence, the T-cell surface membrane protein binding to the virus, have been suggested to inhibit infection. In view of the uncertainties associated with the infection, it becomes important to provide for alternative avenues for vaccines to be developed to provide protection from infection. Perhaps even more importantly, it becomes necessary to find effective therapeutic modalities against HIV and selected retroviruses causing disease in man.

Relevant Literature

Specific interactions between CD4 and the HIV envelope protein are reported by Dalgleish et al., Nature (1984) 312:763-767; Klatzmann et al., Nature (1984) 312:767-768; McDougal et al., Science (1986) 231:382-385. Cleavage of the envelope protein precursor gp160 to gp120/gp41 and transport to the viral membrane or to the plasma membrane of infected target cells is described by Chakrabarti et al., Nature (1986) 320:535-537 Hu et al., Nature (1986) 320:537-540; Robey et al., Science (1985) 228:593-595; and Veronese et al., Science (1985) 229:1402-1405. Fusion leading to productive infection may occur within endocytosed vacuoles (Maddon et al., Cell (1986) 47:333-348) or at the plasma membrane (Stein et al., Cell (1987) 49:659-668). Subsequently, in an envelope protein-dependent manner, fusion may occur between infected and uninfected target cells leading to multinucleated giant cell formation and cell death (Lifson et al., Science (1986) 232:1123-1127; Lifson et al., Nature (1986) 323:725-728). For a general review of retroviral envelope proteins, see Weiss et al., eds. (1984) The Molecular Biology of Tumor Viruses. 2nd edition, Vol. I: RNA Tumor Viruses. Cold Spring Harbor, NY: Cold Spring Harbor Laboratory.

In studies with Sendai virus (a paramyxovirus) and influenza virus (an orthomyxovirus), in the absence of endoproteolytic cleavage of the envelope protein precursor, virions are produced but are not infectious; in the case of Sendai, giant cell formation no longer occurs. (Homma and Ohuchi, J. Virol. (1973) 12:1257-1265; Scheid and Choppin, Virology (1974) 57:475-490; Klenk et al., Virology (1975) 68:426-439; Lazarowitz and Choppin, Virology (1975) 68:440-454; Scheid and Choppin, Virology (1976) 69:265-277.)

RIP7 has a complete HIV retrovirus (Fisher et al. Nature (1986) 320:367-371; Feinberg et al., Cell (1986) 46:807-817).

The similarity of structure between the glyco-proteins of the paramyxoviridae and orthomyxoviridae with retroviridae is suggested by White et al., Quart. Rev. Biophys. (1983) 16:151-195. See also Wilson et al., Nature (1981) 289:366-373.

Novel DNA constructs are provided comprising DNA sequences for in vitro gp160 mutagenesis, the sequence of the mutagenized HIV gp160, constructs comprising the mutagenized HIV gp160, the expression products thereof, methods of preparing the expression products, by themselves or in conjunction with other proteins, as well as use of the products for investigation, as vaccines, and as a probe for investigation of the endoproteolytic cleavage of gp160, the protease(s) involved, and inhibitors thereof.

## DESCRIPTION OF THE SPECIFIC EMBODIMENTS

Methods and compositions are provided to help in the understanding of the etiology of the disease, called AIDS, associated with HIV, for use as a vaccine, as a probe for therapeutics, and the like. The methods and compositions employ mutagenesis of the HIV glycoprotein to prevent endoproteolytic cleavage of the glycoprotein gp160, while allowing for otherwise faithful assembly of the virus. By modifying the nucleic acids in the region of the putative endoproteolytic processing site, so that the polyprotein gp160 may not be processed, a mutated virus may be obtained, which is non-infectious, but which displays substan tially the same epitopes as the wild-type. Further, this mutated gp160 may be used to locate the intracellular protease(s) normally involved in proteolysis of gp160. Once located, inhibitors of these proteases may be found or designed. Such protease inhibitors may then be assessed for clinical efficacy against infection by HIV.

The polyprotein glycoprotein gp160 is characterized by having the amino acid sequence

```
        ← gp120                    gp41 →
I  A  P  T  K  A  K  R  R  V  V  Q  R  E  K  R  A  V  G  I  V  G  A
   ↑        ↑  ↑  ↑                    ↑
```

near the endoproteolytic cleavage site dividing the resultant subunits, gp120 (left) and gp41 (right). The arrows indicate potential tryptic cleavage sites. The DNA encoding the amino acids of the cleavage site region may be modified in a wide variety of ways. Preferably, the amino acids of the cleavage site region are substituted, so as to no longer be subject to endoproteolytic cleavage, where one (or all) of the amino acids associated with recognition may be removed, or one or more amino acids introduced between the amino acids associated with the recognition site. Thus, deletions, insertions, or substitutions of the encoding DNA may involve one or more base pairs, as long as an open reading frame is preserved.

The mutations may involve at least one codon, and usually not more than about 8 codons, more usually not more than about 6 codons. Conveniently, one may provide for a deletion and insertion, where a single base pair is removed in or immediately prior to the codons encoding the cleavage site, so as to change the reading frame, so long as the changed reading frame does not include a stop codon, and a base pair is inserted afterwards to restore the proper reading frame.

Alternatively, base pairs may be substituted, by transitions or transversions, where the mutation is not a silent mutation. Preferably, the change in sequence results in the retention of the same number of amino acids, but results in a change in the amino acid sequence of the cleavage site. For example, since the cleavage site involves positively charged amino acids, other amino acids could be used which are neutral, such as the aliphatic amino acids, which include the nonpolar amino acids, G, A, P, V, L, and I; the heteroatom containing amino acids, C, M, S, T, N, Q; the negatively charged amino acids, D, E; and the aromatic amino acids, F, H, W, Y. Alternatively, the sequence of the different positively charged amino acids in the vicinity of the cleavage site could be altered. Desirably, the modification will result in a change in sequence which does not interfere with the proper folding of the gp160 protein for mimicking gp120 and gp41.

For convenience, one may wish to introduce one or more novel restriction endonuclease sites in the region of the altered base pairs. The presence of such restriction sites provides for a number of procedural advantages. First, one can quickly determine whether the desired mutation is present by restriction mapping of the fragments employing gel electrophoresis. In addition, if one has two restriction sites adjacent to and bordering the mutated region, that region may be easily exchanged with other sequences, by digesting the gene and rejoining the resulting two arms with an adapter. Thus, there would be the opportunity to vary the amino acids at the cleavage site and to optimize the changes allowing for use of the subject proteins for investigation of endogenous proteases or as vaccines.

The subject DNA compositions may be prepared in a variety of ways. Conveniently, a naturally occurring gp160 gene may be isolated from a particular HIV strain and inserted into a single strand phage vector for site-directed oligonucleotide-mediated mutagenesis. By employing a "double primer" method, where the second primer has the mutagenized oligonucleotide sequence, a mutagenized gene may be obtained. Alternatively, unique restriction sites bracketing the codons encoding the cleavage site region may be introduced into the wild-type DNA sequence in a similar manner; these may then be digested and the resulting two ends joined by an adapter encoding the desired mutated sequence. The particular mode of introducing the mutagenic site, so as to change the amino acid sequence of the endoproteolytic cleavage site is not critical to this invention, so long as the gene may be mutagenized and the mutagenized strand retains an open reading frame.

Conveniently, where oligonucleotide-mediated in vitro mutagenesis of single-stranded phage is employed, the heteroduplexes which are obtained may be transformed into repair-deficient hosts (mutL) and propagated on recA lawn cells, selecting for plaques. The transfected DNA may then be isolated and analyzed for the presence of the mutagenized strand. The replicative form of the phage may then be isolated, the gene excised, cloned and analyzed to establish the presence of the desired lesions. The resulting gene will usually encode at least about 95%, more usually at least about 98% of the amino acids of the natural gp160.

The mutated gp160 gene may then be transferred to a vector, particularly a non-lytic vector, for transfection into a mammalian host. Any convenient mammalian host may be employed which will provide for effective expression of the gene. In addition, it will be desirable to have glycosylation of the expressed product, so as to most closely approximate the post-translational processing of the natural protein. Convenient cell lines include COS-1 cells, CHO cells, HeLa cells, etc.

The expression product may then be used as a vaccine. The expression product may be prepared in any convenient host, particularly mammalian host, although prokaryotic and eukaryotic microbial hosts may be employed, e.g. E. coli, yeast, e.g. S. cerevisiae, Kluyveromyces, e.g. lactis or fragilis, Neurospora, Aspergillus, etc. The protein may be secreted or retained in the cytoplasm, isolated in accordance with conventional ways, purified to substantially complete purity (>95% purity), and may then be used for vaccination.

Preferably, for detection of mutations which block endoproteolytic cleavage, the mutating oligonucleotide will be used to modify a substantially intact virus capable of replication, i.e., having functional pol, gag, sor, tat and trs genes, and also having an intact long-terminal repeat (LTR). This can be achieved by introducing the DNA fragment which includes the mutagenized portion of the env gene into the wild-type HIV viral genome, where the viral genome is part of a vector which may cause replication of the HIV genome whether or not the produced HIV virions are infectious. A number of strains of HIV have been inserted into vectors having functional independent replication systems for transfection into mammalian cells. Usually, the vector will have a viral replication system, such as the SV40 replication system, adenovirus replication system, bovine papilloma virus replication system, or the like.

A large number of vectors have been developed which can be used for insertion of the HIV sequence into the vector and provide for stable replication in a mammalian host. In addition to providing replication and expression, the vector may have other functions, such as a replication system functional in a prokaryote, particularly E. coli, and one or more markers which allow for selection in the prokaryotic host and/or eukaryotic host.

Upon transfection of the plasmid into normal human or other mammalian cells or cell lines, the plasmid vector uses the functional replication system to result in HIV replication, RNA processing, protein translation and assembly. However, with the mutated gp160 protein, when the virus is transfected into a T-lymphoblastoid cell line or the non-T-cell eukaryotic host is co-cultured with a T-lymphoblastoid cell line or normal T-helper cells, the pattern of secondary infection and giant cell formation observed with infectious HIV is absent. An initial reverse transcriptase peak in the supernatant is observed 2 days after transfection, similar to the parent wild-type HIV. Thus, while the mutated virion appears to lack the capacity to infect permissive cells as well as the fusogenic potential for giant cell formation between infected and uninfected cells, it is able to perform the other functions associated with replication and assembly of the virion.

The subject virion can be used particularly as a vaccine. Since the proteins involved with the envelope and capsid are the same as the wild-type proteins, except for the alteration in the gp160 endoproteolytic cleavage site, the epitopes presented to the host immune system will be, for the most part, the same as those presented by the wild-type virus. To that extent, the host immune system may be able to respond to the attenuated virus by mounting both a cellular and humoral response, since the proteins of the attenuated virus will be digested by antigen presenting cells whereby both CD4 and CD8 cells will become involved. Thus, a strong immune response will be achieved.

Transfected cells may be used as a source for growth of the attenuated virus. The cells may be grown, transfected, and the virus allowed to replicate and multiply in the cells. The virus may then be isolated and substantially purified, being free of substantially all of the nucleic acid, proteins and sugars associated with the host cells. The virus may then be used as a vaccine.

Alternatively, the subgenomic region encoding the mutated gp160 envelope may be isolated from the rest of the virus within a high-expression eukaryotic expression system, for instance, a deletion mutant of HIV retaining the LTR, envelope, and tat regions, but lacking functional gag and pol genes, e.g. HXB2, R/CP1, etc. Such a construct when transfected into human cells, e.g. HeLa cells, will drive high level expression of the mutated gp160 in the absence of viral particles. Once purified, the mutated protein may serve to elicit a neutralizing antibody response.

As a vaccine, the virus may be administered in a variety of ways, including subcutaneous, intravascular, intraperitoneally, or the like. The virus may be formulated in a physiologically acceptable medium, such as saline, phosphate buffered saline, or the like. The concentration may be varied widely, depending upon the manner of administration, for example the number of injections, the sites of injection, or the like. The amount of virus injected will be determined empirically depending on the observed response.

The vaccine may be administered in conjunction with various adjuvants to enhance the immune response. Various adjuvants are well known, and one may be favored as compared to another as to the manner of administration, the vaccine, and the like.

In addition to being used as a vaccine, the mutated gp160 may also be used as a probe to identify protease(s) capable of endoproteolytic cleavage of wild-type gp160. These proteases are endogenous products of the virally-infected host cells and are probably compartmentalized within a discrete stage of the secretory pathway. As gp160 passes through this stage, during the course of intracellular viral maturation, the heterodimer gp120/gp41 is formed after cleavage. The mutated gp160 may bind to the protease(s) but not be cleaved, providing a competitive inhibitor for wildtype gp160.

By cotransfecting host cells which wild-type viral constructs and mutated viral constructs which may act as a competitive inhibitor for the endoprotease(s), the endoprotease(s) may be identified in a lysate, which may then be fractionated. The mutated gp160 may be modified to provide for affinity binding to the endoprotease(s), where antibodies to gp160 may be used to isolate the mutated gp160/endoprotease conjugate.

The following examples are offered by way of illustration and not be way of limitation.


## EXPERIMENTAL

RIP7 carries a biologically active HIV proviral clone (HXB2) within the parent vector SP65gpt; in addition, it encodes the SV40 origin for propagation in COS-1 cells, the xgpt gene for dominant selection in eukaryotic cells, and the pBR ori and the amp resistance gene for propagation in E. coli. When it is transfected into COS-1 cells, human T-cell lines or HeLa cells, it gives rise to infectious cytopathic HIV virions. Replication, RNA processing, protein translation, and assembly of the virions proceed in a fashion indistinguishable from that of HIV itself. Reverse transcriptase (RT) activity is found to peak in the supernatant by day 2 and to then subside. The co-culture of $CD4^+$ VB cells (a human T-lymphoma cell line) with RIP7-transfected COS-1 cells results in secondary infection, further rounds of viral replication, and progressive increases in supernatant RT activity. Concomitantly, giant cells form. These multi-nucleated cells are readily visualized and quantitated under lower power phase contrast microscopy. They represent fusions between uninfected VB cells and infected VB cells and/or RIP7-transfected COS-1 cells. When uninfected VB cells are stained with a nuclear dye Hoechst 33342 and then mixed with RIP7-transfected COS-1 cells, multi-nucleated giant cells form which have incorporated (blue) VB nuclei into a cytoplasm which counter-stains (yellow) with fluoresceinated anti-HIV antibodies.

RIP7/mut10 carries a mutated gp160 subregion generated by oligonucleotide-mediated site specific mutagenesis, as described infra. The mutation removes a tryptic-like cleavage site from the wild-type gp160 in the vicinity of the normal endoproteolytic cleavage site separating gp120 and gp41. The same mutation also introduces a novel cleavage site for chymotrypsin, absent in wild-type gp160.

When RIP7/mut10-transfected COS-1 cells are co-cultured with VB cells, the pattern of secondary infection and giant cell formation observed with RIP7 is absent. An initial RT peak in the supernatant occurs 2 days after transfection, similar to the wild-type RIP7. In the presence of co-cultured VB cells, however, no progressive rise in supernatant RT activity is observed. Furthermore, no giant cells are seen to form.

To confirm that the oligonucleotide-directed lesions introduced into RIP7/mut10 are responsible for the loss of biologic activity, a number of control evaluations were pursued. After transfection into COS-1 cells, the proviruses of RIP7 and RIP7/mut10 were found to be replicated with fidelity. A novel EcoRI site of RIP7/mut10 (introduced by the mutated base pair changes) was retained when extrachromosomal DNA was analyzed after Hirt extraction.

The transfection efficiency of COS-1 cells with each construct was analyzed by immunofluorescence. Using a polyclonal antiserum selected for reactivity against envelope protein epitopes, the transfection efficiency of COS-1 cells with RIP7 and RIP7/mut10 was found to be low (less than 1%) but roughly equivalent. When total cellular RNA was prepared from RIP7 and RIP7/mut10-transfected COS-1 cells and analyzed by semi-quantitative dot blots, each was found to yield similar transcriptional yields of 5′ and 3′ mRNAs. When analyzed by Northern blots, these mRNAs were found in both cases to be processed identically.

HIV-encoded protein was detected in COS-1 cells after transfection with RIP7/mut10 with cytoplasmic staining intensity and distribution similar to that found in RIP7-transfected COS-1 cells. The expression of the HIV envelope protein on the surface of transfected COS-1 cells was evaluated using a human monoclonal antibody specific for an epitope of gp41 (also found on gp160). Cells transfected with RIP7 showed bright surface fluorescence with this antibody; those transfected with RIP7/mut10 showed weaker fluorescence, well above that of background. Both RIP7 and RIP7/mut10-transfected COS-1 cells also expressed similar amounts of cell surface gag, as assayed with a monoclonal anti-p25 antibody.

The ability of each construct to yield fully mature viral particles was assessed in two ways. As previously discussed, both are found to produce a similar amount of RT activity into the supernatant of transfected COS-1 cells peaking at day 2. This RT activity was found to be associated with mature, morphologically similar viral particles as shown by direct electron microscopy.

To rule out the possibility that RIP7/mut10 may carry a defect in the sub-genomic regions gag or pol, complementation experiments were carried out with the HIV mutants HXB2 RIPI and HXB2 R/CPI. HXB2 RIPI bears a point mutation which interrupts the reading frame of the pol gene; HXB2 R/CPI bears a deletion spanning the gag and pol regions of HIV. Both have a wild-type 3′ end including the env subgenomic region. When transfected into COS-1 cells, neither is found to result in the release of RT activity or, upon addition of VB cells, in the generation of giant cells. If, however, either is co-transfected into COS-1 cells along with RIP7/mut10, RT activity is released and giant cells do form. Thus, the wild-type 3′ region of each has complemented the envelope mutation of RIP7/ mut10; reciprocally, the 3′ gag and pol regions of RIP7/mut10 are functional.

To show that the defect in the RIP7/mut10 is associated with the introduced lesions, two experiments were carried out. First, Western analysis was used to evaluate envelope protein processing in COS-1 cells transfected with RIP7 or RIP7/mut10. Two different antibodies were employed: a polyclonal anti-HIV antiserum reactive with both gp160 and gp120, and a monoclonal anti-gp41/gp160 antibody unreactive with gp120. As observed, gp160 is recognized specifically by the monoclonal antibody in COS-1 cells transfected with RIP7, as well as in H9 cells infected with HXB2. In RIP7-transfected COS-1 cells, gp120 is identified by the polyclonal anti-HIV antiserum and gp41 is detected by the monoclonal anti-gp41/gp160 antibody. In COS-1 cells transfected with RIP7/mut10, however gp160 is produced, but gp120 and gp41 are not. The mutation introduced into RIP7/mut10 thus prevents the endoproteolytic cleavage of gp160.

The mutation in RIP7/mut10 creates a new cleavage site for chymotrypsin, not found in the cleavage site of RIP7. To further demonstrate that endoproteolytic cleavage of gp160 is necessary for the activation of fusogenic activity, chymotrypsin was added. After empirically determining that RIP7-transfected COS-1 cells would fuse with VB cells in serum concentrations as low as 0.16% and chymotrypsin concentrations ranging between 0-32 μg/ml (in 0.5% serum) RIP7/mut10-transfected COS-1 cells were then cocultured with VB cells in 0.5% serum and subjected to constant proteolysis with incremental concentrations of chymotrypsin. In the presence of 1-4 μg/ml chymotrypsin, giant cells formed in low frequency. These giant cells were morphologically similar to those formed between RIP7-transfected COS-1 cells and VB cells: they consisted of multiple pyncnotic nuclei surrounded by both a ballooning cytoplasm and by rosetted but unfused VB cells. As evidenced by double-color fluorescence, the nuclei were contributed by VB cells (previously stained blue with the nuclear dye Hoechst 33342) and the cytoplasm counter-stains yellow with fluoresceinated antibodies against HIV. Therefore, once the endoproteolytic cleavage of the gp160 is restored, the fusogenic activity of the mutated RIP7/mut10 virions is also restored.

It is evident from the above results that non-infectious HIV viruses can be produced for use as vaccines, where the virus remains highly immunogenic, maintains substantially the same conformation of the natural virus, but lacks infectivity and fusogenic capability. In addition, the subject compositions may be used as probes for structural/functional correlates in and about the vicinity of the endoproteolytic cleavage site of gp160. The subject compositions may also be used as probes for the isolation and characterization of the intracellular proteases involved in the endoproteolytic cleavage of gp160. Identification of these proteases may, in turn, permit the systematic evaluation of specific inhibitors of their function. Finally, the subject compositions may also be used for the identification and isolation of immune (T-and B-) cells specifically reactive against epitopes of HIV, to evaluate cells which may lead to the description of effective cellular and/or humoral defenses against HIV infection.

Experimental Procedures

Plasmids

pHXB2gpt contains the viral insert of HXB2D (Shaw et al., Science (1984) 266:1165-1171) and the E. coli xgpt gene each oriented in the same transcriptional orientation in pSP65 (Fisher et al., Nature (1986) 320:367-371). RIP1 and RIP7 were constructed by partial digestion of pHXB2gpt with EcoRI followed by incubation with the Klenow fragment of DNA polymerase I in the presence of nucleoside triphosphates and reclosure with T4 DNA ligase. In HXB2 RIP1, the EcoRI site at map position 5028 (numbering system of Ratner et al., Nature (1984) 313:277-284), was removed creating a point mutation in the polregion; in RIP7, an EcoRI site outside of the HXB2 insert was removed. To construct the gag/pol deletion mutant HXB2 R/CPI, pHXB2gpt was grown in the dam⁻ host GM161, cut completely with ClaI, and then partially with EcoRI; isolates cut at the EcoRI site at position 5323 were filled in with Klenow and closed with T4 DNA ligase. The sequencing vector pBSM13 was obtained from Stratagene Cloning Systems, San Diego, CA 92121. M13mp18 and M13mp19 have been described (Norrander et al., Gene (1984) 26:101-106). All cloning manipulations followed standard procedures (Maniatis et al., (1982) Molecular Cloning: A laboratory manual. Cold Spring Harbor, NY: Cold Spring Harbor Laboratory).

Antibodies

IB8 is a human monoclonal antibody against an epitope of gp41 (and thus, gp160), available from Genelabs, Inc., Redwood City, CA, and used at a concentration of 5 μg/ml IgG in immunofluorescence assays and in Western analyses. GB is a high-titer human antiserum against HIV, used at 1:50-1:500 for immunofluorescence assays and 1:750 on Westerns. Anti-p25 (Litton Bionetics) is a mouse monoclonal antibody against the HIV gagsubunit p25, used at 2 μg/ml IgG for immunofluorescense. Fluoresceinated second-stage antibodies against mouse and human IgG were purchased from Cappell.

Cell Lines

The T-lymphoblastoid cell lines H9 and H9/HXB (Popovic et al., Science (1984) 224:497-500) and VB (Lifson et al., Science (1986) 232:1232-1237) were maintained as described. The SV40-transformed African green monkey cell line COS-1 (Gluzman, Cell (1980) 23:175-182) was grown at 37°C in a 5% CO₂ atmosphere in DME medium supplemented with 10% fetal calf serum (FCS).

Oligonucleotide Site-Directed Mutagenesis

The target for site-directed mutagenesis, a 2.6 kB SalI/BamHI envelope fragment from pHXB2gpt was moved into M13mp18 to create M13mp18/env. The mutating oligonucleotide,

```
                                        EcoRI
30-mer   GTGGTGCAGGGAGAA|GAATTC|GCAGTGGGA
          V   V   Q   G   E   E   F   A   V   G
```

was a 30-mer designed according to the general principles of Zoller and Smith (Meth. Enzymol. (1983) 100: 468-500). Computer-assisted homology searches were carried out using the HXB2 and M13 sequences pulled from the NIH data bank. The oligonucleotide was synthesized by the solid-state phophodiester procedure and purified as described (Lo et al., Proc. Natl. Acad. Sci. USA (1984) 81:2285-2289). Site-directed mutagenesis on the single-stranded progeny of M13mp18/env was carried out by the "double primer" method (Zoller and Smith, DNA (1984) 3:479-488) using the universal M13 sequencing primer and the mutating oligonucleotide. Heteroduplexes were then transformed into the repair-deficient (mutL::Tn10) strain HB2154 (Carter et al., Nucleic Acids Res. (1985) 13:4431-4443) and propagated in recA⁻ JM109 lawn cells (Yanisch-Perron et al., Gene (1985) 33:103-119). 220 plaques resulting from the transfected DNA were grown as colonies on L-plates, transferred to nitrocellulose, and probed with the ³²P-kinased mutating oligonucleotide as previously described (Wallace et al., Nucleic Acids Res. (1981) 9:879-894). All colonies hybridized at 55°C; 31 hybridized at 58°C, of which 25 (12%) gave strong signals at 70°C. Replicative forms from these colonies were isolated and digested with SalI, BamHI and EcoRI; 9 (4%) showed the insertion of a novel EcoRI site dividing the

Sall/BamHI envelope fragment into two fragments of approximate MW 1.9 kB and 0.7 kB. From these isolates, 0.5 kB BglII/HindIII fragments, encompassing the inserted EcoRI site near the coding region for the endoproteolytic cleavage site, were subcloned into BamHI/HindIII-cut pBSM13 vectors for bidirectional nucleotide sequence analysis. These constructs were manipulated as described by the manufacturer, using dideoxy chain termination reactions (Sanger et al., J. Mol. Biol. (1980) 143:161-178).

## Eukaryotic Cell Transfections

Transfections of COS-1 cells for DNA, RNA, or protein analysis, or for viral particle isolation were carried out as described (Lopata et al., Nucleic Acids Res. (1984) 12:5707-5715). COS-1 cells were seeded at a density of $2 \times 10^6$ cells/100 mm plate, $2 \times 10^5$ cells/well in a 24-well plate (Costar, Cambridge, MA, 02139), or $10^4$ cells/well in an 8 chamber Lab-Tek plate (Miles Scientific, Naperville, IL, 60566), transfected with 10 μg of plasmid DNA/ml for 6-12 hours, and shocked with 10% DMSO for 2 min prior to recovery in DMEM with 10% FCS.

## Nucleic Acid Analysis

Unintegrated HIV DNA was prepared from RIP7 and RIP7/mut10-transfected COS-1 cells using a modification (Stein et al., Cell (1987) 49:659-668) of a previously-described procedure (Hirt, J. Mol. Biol. (1967) 26:5707-5717). 20 μg/lane was electrophoresed on a 0.8% agarose slab gel, transferred to nitrocellulose, and probed with $^{32}$P-labelled HIV DNA. All probes were isolated from low-melting point agarose after restriction endonuclease digestion and nick-translated according to established procedures (Maniatis (1982) supra). Following hybridization, filters were washed once in 2x SSC (1x SSC = 0.15M NaCl, 0.015 M sodium citrate), 0.5% SDS for 5 min at room temperature, once in the same buffer for 5 hrs at 50°C, and then once in 0.1x SSC, 0.1% SDS for 1 hr at 50°C. Hybridizing bands were detected by autoradiography using Kodak XAR film.

RNA from transfected COS-1 cells was isolated by the hot phenol extraction procedure (Queen and Baltimore, Cell (1984) 33:741-748) and processed for Northern analysis and for semiquantitative dot blots as described (Feinberg, Cell (1986) 46:807-817).

## Reverse Transcriptase Assay

Culture supernatants were processed for reverse transcriptase activity using an assay adopted from Hoffman et al., (Virology (1985) 147:326-335). Supernatants were first centrifuged for 5 min at 2000 rpm to remove cells and cell debris. To pellet viral particles, 1.5 ml was transferred to a microfuge tube and centrifuged for 30 min at 15,000 rpm, 4°C, in a microcentrifuge. The pellets were resuspended in 50 μl of a reaction mixture containing 50 mM Tris-HCl (pH 8.0), 5 mM DTT, 20 mM MgCl₂, 150 mM KCl, 0.5 mM EGTA, 0.05% Triton-100, 25 μg/ml poly rA:oligo dT 12-18 primer, and 5-10 μCi $^3$H-TTP (New England Nuclear, NET 221X), and incubated at 37°C for 45 min. Reactions were stopped by adding 0.4 ml of 100 mM sodium pyrophosphate, 10 mM EDTA, and 5 μg/ml tRNA, and 1.0 ml of 10% trichloroacetic acid. After 10 min on ice, reactions were filtered through a glass filter (S&S #30, 25 mm) and washed 3 times with cold 1 M HCl, 10 mM sodium pyrophosphate, and once with 70% ethanol. Once dried, each filter was placed in a scintillation vial with 3 mls of Omnifluor and counted. Positive controls included culture supernatants from the HIV-producing cell line H9/HXB. Negative controls included (1) the substitution of 5 mM EDTA for 20 mM MgCl₂ in the reaction mix, and (2) the use of culture supernatants from uninfected H9 cells or, as appropriate, from sham-transfected COS-1 cells.

## Protein Analysis

HIV envelope proteins synthesized in transfected COS-1 cells were analyzed by Western blot, using a modification of established methods (Burnette et al., Anal. Biochem. (1981) 112:195-203; Towbin et al., Proc. Natl. Acad. Sci. USA (1979) 76:4350-4354). 48-72 hours after transfection, cells were washed once with phosphate buffered saline and lysed by repeated vortexing at a concentration of $10^7$/ml, in a buffer containing 1% Triton X-100, 0.5% sodium deoxycholate, 250 mM NaCl, 25 mM Tris-HCl (pH 7.5), and 5 mM EDTA. Aliquots corresponding to $5 \times 10^5$ cells were resolved by SDS-PAGE on 10% polyacrylamide gels and transferred to nitrocellulose (S&S, 0.45 μ) in blotting buffer (190 mM glycine, 20% methanol, and 25 mM Tris-HCl, pH 8.0) for 8 hrs at 250 mA. The membranes were blocked with 1% gelatin in TBS (500 mM NaCl, 20 mM Tris-HCl, pH 7.5) and then incubated as strips for 10-12 hrs at room temperature with the indicated antibodies or with nonimmune serum. After 3 washes in TBS, the strips were counterstained with alkaline phosphatase-conjugated goat anti-human IgG (Promega Biotech) and reacted with nitroblue tetrazolium and 5-bromo-4-chloro-3-indolyl phosphate in 100 mM NaCl, 100 mM Tris-HCl (pH 9.5) and 5 mM MgCl₂.

## Electron Microscopy

For direct electron microscopic visualization of virions produced by RIP7 or RIP7/mut10-transfected COS-1 cells, culture supernatants (40 ml) were harvested 48 hrs post-transfection. Cell debris was pelleted by centrifugation at 2000 rpm for 20 min. The supernatant from this spin was then centrifuged at 23,000 rpm for 2 hrs at 4°C in a Beckman SW27 rotor. The viral pellet from this spin was fixed in 3% glutaraldehyde, embedded in osmium tetroxide, and prepared by thin sectioning for electron microscopy, using established procedures.

Chymotrypsin Reversal of RIP7/mut10

For analysis of the effect of chymotrypsin on the biologic activity of RIP7/mut10, it was first empirically established that giant cell formation would occur in low serum conditions. COS-1 cells were transfected in 24-well microtiter plates with either RIP7 or RIP7/mut10 and allowed to recover in DMEM with no serum or with FCS concentrations ranging in doubling increments from 0.16% to 10%. Two days after transfection, $10^6$ VB cells (washed 3 times in serum-free DMEM)/well were added. Within 3 days, multinucleated giant cells were visualized under phase-contrast microscopy and counted. FCS concentrations as low as 0.16% were found to support giant cell formation between RIP7- transfected COS-1 cells and VB cells; RIP7/mut10-transfected COS-1 cells and VB cells did not form giant cells at any serum concentration

Accordingly, in chymotrypsin reversal experiments, COS-1 cells were supplemented with DMEM and 0.5% FCS after transfection. On days 1, 3, and 5 after transfection, chymotrypsin (from a freshly-made stock, 10 mg/ml in 0.1 mM HCl) was added to the indicated concentration, ranging from 0 μg/ml to 128 μg/ml. $10^6$ VB cells/well were co-cultured with the COS-1 cells 2 days after transfection. By day 5, giant cells were visualized under low power phase-contrast microscopy, counted, and processed for immunofluorescence.

Immunofluorescence

Immunofluorescence assays were carried out on cells fixed in 10% $H_2O$/acetone for detection of intracellular antigens or, for visualization of surface antigen, on live COS-1 cells adherent to Lab-Tek microtiter wells. Nonspecific background was reduced by pre-incubation of slides with 10 μg/ml goat anti-mouse IgG for 30 min at room temperature prior to the successive additions of specific antibody and fluoresceinated second antibody. In some cases cells were also counterstained with Evans blue.

The double-color staining of multinucleated giant cells was carried out as follows. COS-1 cells were transfected in 15 mm Lab-Tek wells with RIP7 or RIP7/mut10 DNA and then allowed to recover in DMEM with 0.5% FCS, with or without 2 μg/ml chymotrypsin. Two days after transfection VB cells were incubated with the nuclear dye Hoechst 33342 (5 mM) for 30 min at 37°C, washed 3 times in DMEM, and added at a concentration of $10^5$/well. On day 6, the cells were fixed on Lab-Tek slides in 10% $H_2O$/acetone, stained with the polyclonal antiserum GB and fluorescein anti-human Ig, and visualized on a Zeiss Universal photomicroscope equipped for epifluorescence. The same fields were photographed with filters to observe either blue (Hoechst 33342) or green (fluorescein) fluorescence; under the exposure conditions used, emissions from the latter fluorochrome appear yellow.

As demonstrated, novel DNA sequences are provided which provide for expression products which may find use in the production of vaccines against AIDS. The expression products provide for a protein which is cross-reactive with HIV-1 or for an HIV-1 virion which lacks infectious capability and is unable to provide for syncytial fusion of cells. Novel expression vectors are provided which serve to transform cells for the production of the vaccines. The subject DNA sequences and the expression products also find use in the study of the HIV virus in providing for inhibitors of the enzymatic cleavage of gp160.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced.

**Claims**

1. A DNA sequence encoding substantially the entire HIV gp160 protein lacking a functional endoproteolytic cleavage site.

2. A DNA sequence according to claim 1, wherein said DNA sequence is mutated to substitute two different amino acids for the natural cleavage site.

3. A DNA vector comprising an origin of replication functional in prokaryotes, a marker for selection in prokaryotes, and a DNA sequence encoding substantially the entire HIV gp160 protein lacking a functional endoproteolytic cleavage site.

4. A DNA vector according to claim 3, comprising a transcriptional initiation region and a transcriptional termination region functional in mammalian cells, and wherein said DNA sequence is between said initiation region and termination region and under the transcriptional regulation of said initiation region.

5. A DNA vector comprising an origin of replication functional in eukaryotes, a marker for selection in eukaryotes, and a DNA sequence encoding substantially the entire HIV gp160 protein lacking a functional endoproteolytic cleavage site.

6. A DNA vector according to claim 5, comprising a transcriptional initiation region and a transcriptional termination region functional in mammalian cells, and wherein said DNA sequence is between said initiation region and termination region and under the transcriptional regulation of said initiation region.

7. A mutated HIV virion genome comprising a DNA sequence encoding substantially the entire HIV gp160 protein lacking a functional endoproteolytic cleavage site.

8. A mutated HIV virion genome according to claim 7, wherein said DNA sequence is mutated to substitute two different amino acids for the natural processing signal.

9. An HIV gp160 encoding DNA sequence comprising the partial sequence bridging the processing signal consisting of C A G G G A A G A A T T C G C A G T G, wherein G A A T T C, replaces A A A A G A.

10. A method for producing a vaccine to protect a human host against the HIV virus, said method comprising:
growing a mammalian cell competent for replicating and expressing the HIV virion transformed with a mutated HIV virion genome comprising a DNA sequence encoding substantially the entire HIV gp160 protein lacking a functional endoproteolytic cleavage site.

11. A method according to claim 10, wherein said HIV virus is joined to a replication system functional in said cell to form a plasmid.

12. An HIV vaccine comprising the expression product of DNA sequence according to claim 1.

13. A method according to claim 10, claim 11 or claim 12, wherein the HIV gp160 encoding DNA sequence comprises the partial sequence bridging the processing signal consisting of C A G G G A A G A A T T C G C A G T G, wherein G A A T T C, replaces A A A A G A.

## Claims for the following Contracting States: ES, GR

1. A method for producing a vaccine to protect a human host against the HIV virus, said method comprising:
growing a mammalian cell competent for replicating and expressing the HIV virion transformed with a mutated HIV virion genome comprising a DNA sequence encoding substantially the entire HIV gp160 protein lacking a functional endoproteolytic cleavage site.

2. A method according to claim 1, wherein said cleavage site is mutated as a result of at least one base pair substitution.

3. A method according to claim 1 or claim 2, wherein said DNA sequence is mutated to substitute two different amino acids for the natural processing signal.

4. A method according to any one of the preceding claims, wherein said HIV virus is joined to a replication system functional in said cell to form a plasmid.

5. A method according to any one of the preceding claims, wherein the HIV gp160 encoding DNA sequence comprises the partial sequence bridging the processing signal consisting of C A G G G A A G A A T T C G C A G T G wherein G A A T T C replaces A A A A G A.

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| P,X | CELL, vol. 53, no. 1, April 8, 1988 (Cambridge, Mass., USA)<br><br>J.M. MC CUNE et al. "Endoproteolytic Cleavage of gp 160 is Required for the Activation of Human Immuno-deficiency Virus"<br>pages 55-67<br><br>　　* Totality *<br><br>--- | 1,3,5, 7 | C 12 N 15/00<br><br>C 07 H 21/04<br><br>A 61 K 39/21 |
| X | EP - A1 - 0 245 136 (TRANSGENE S. A. et al.)<br><br>　　* Claims 1,4-6,14 *<br><br>--- | 1,3,5, 7 | |
| P,X | JOURNAL OF VIROLOGY, vol. 62, no. 9, September 1988 (Washington DC)<br><br>P.W. BERMAN et al. "Expression of Membrane-Associated and Secreted Variants of gp 160 of Human Immunodeficiency Virus Type 1 In Vitro and in Continuous Lines"<br>pages 3135-3142<br><br>　　* Pages 3135-3137, 3140 *<br><br>--- | 1,7 | |
| P,A | EP - A2 - 0 272 858 (REPLIGEN CORPORATION)<br><br>　　* Abstract *<br><br>---- | 1 | |

**TECHNICAL FIELDS SEARCHED (Int Cl 4)**

C 12 N

C 07 H

A 61 K

The present search report has been drawn up for all claims

| Place of search | Date of completion of the search | Examiner |
|---|---|---|
| VIENNA | 15-06-1989 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82